# EUROPEAN PATENT APPLICATION

(11) **EP 4 817 653 A1**
(43) Date of publication of application: **07.10.2026**
(21) Application number: 25168277.9
(22) Date of filing: 03.04.2025
(51) Int. Cl.: A61M 5/145

(54) **A DEVICE FOR EMPTYING OR FILLING PISTON SYRINGES**

(71) Applicant: Laser in motion v/Jesper Frydekjær Floridong, 4200 Slagelse (DK)
(72) Inventor: Burild, Thomas, 4200 Slagelse (DK)
(74) Representative: Larsen & Birkeholm A/S

(57) **Abstract**

A device for emptying piston syringes (30) of the type comprising a piston that can be pressed into a cylinder (36) via a piston rod (37), with a first end provided with a tip (33) for attaching a needle and a second end normally provided with a flange, the device comprising:a syringe section (10) with a fixed part (6) for attachment to a piston syringe cylinder (36, 33) and a movable part (7) for attachment to a piston syringe piston rod (37); wherein the movable part (7) can move in both directions relative to the fixed part (6) so that a syringe (30) fixed in the syringe section (10) can be emptied or filled by moving the movable part (7) relative to the fixed part (6), characterized in that the movable part (7) slides on the fixed part (6) and is attached to one part of a wire (2) that runs along the fixed part (6) in a loop over a first pulley, whereby the movable part (7) moves in opposite directions relative to the fixed part (6) when the wire (2) runs around the pulley (8) clockwise or counterclockwise.

## Description

The present invention relates to a device for emptying or filling piston syringes of the type comprising; a piston that can be pressed into a cylinder with a first end provided with a tip for attaching a needle and a second end normally provided with a flange, the device comprising: a syringe section with a fixed part for attachment to a piston syringe cylinder and a movable part for attachment to a piston syringe piston rod; wherein the movable part can move in both directions relative to the fixed part so that a syringe fixed in the syringe section can be emptied or filled by moving the movable part relative to the fixed part.

The present invention is useful in the field of medical devices, particularly useful in syringe-based drug delivery systems. More specifically, it pertains to a controlled mechanism for emptying or filling a syringe, either partially or fully, to facilitate the precise administration of medication.

### Background of the Invention

In modern healthcare, the administration of medications is a critical procedure that requires precision and control. Syringe-based drug delivery is widely used in hospitals, clinics, and emergency settings to administer various pharmaceutical agents, including anesthetics, antibiotics, pain relievers, and other essential medications. The ability to accurately control the volume and rate of drug delivery is vital to ensuring patient safety, minimizing dosage errors, and optimizing therapeutic efficacy.

Traditionally, manual syringe operation has been the predominant method for drug administration. A healthcare professional manually depresses the plunger of a syringe to deliver the required dose. While this approach is simple and cost-effective, it presents several challenges, particularly when a precise and controlled infusion is required. These challenges include:
Inconsistent flow rate: The force applied by the user can vary, leading to fluctuations in the flow rate of the medication being administered. This inconsistency can be particularly problematic for drugs requiring slow, steady infusion.

Risk of human error: Manual administration is subject to human variability, leading to potential overdoses or underdoses. Fatigue, inattention, or lack of experience can exacerbate these risks.

Incompatibility with long-duration infusions: For extended drug delivery, a healthcare professional must maintain continuous pressure on the plunger, which can be impractical and exhausting over time.

Limited ability to integrate with automated systems: Many modern healthcare settings rely on infusion pumps or electronic drug administration systems. Standard syringes without controlled emptying mechanisms often require manual intervention or separate pumping equipment.

To address these challenges, several attempts have been made to develop controlled syringe mechanisms. These fall into the following broad categories:
Mechanical spring-driven syringes: Some prior art solutions incorporate springloaded mechanisms that provide a consistent force on the plunger. However, these systems lack fine-tuned control over the flow rate and can be affected by variations in spring tension over time.

Motorized syringe pumps: Motorized syringe pumps provide a highly controlled means of drug delivery, often allowing programmable infusion rates. However, these devices are expensive, require external power sources, and can be cumbersome for bedside or mobile use.

Gravity-assisted infusion devices: Some infusion systems rely on gravitational force to control drug flow from a syringe or IV bag. These systems often require precise height adjustments and may not be suitable for all administration scenarios.

Manually adjustable plunger stoppers: Certain syringes feature plunger stoppers that allow incremental drug release, but they still depend on manual operation, limiting their consistency and automation capabilities.

Another significant issue in syringe-based drug administration is the physical strain on healthcare professionals. The repetitive motion required to manually depress syringes, especially during prolonged or frequent administrations, contributes to work-related musculoskeletal disorders. Nurses and other medical staff are particularly susceptible to conditions such as repetitive strain injuries (RSIs) and carpal tunnel syndrome due to the continuous and forceful pressing of syringe plungers. These injuries can lead to chronic pain, reduced dexterity, and even long-term disability, ultimately impacting the efficiency of healthcare services. By introducing a controlled mechanism for syringe emptying, the present invention aims to reduce the physical burden on healthcare workers, improving occupational health and overall workplace safety.

Despite these advances, there remains a need for a syringe-based delivery system that provides controlled, adjustable, and predictable drug administration while minimizing human error and operational complexity. A solution that can be integrated with existing medical workflows, offering enhanced safety and ease of use, would be of significant benefit to healthcare professionals and patients alike.

The present invention seeks to address these shortcomings by introducing a novel system that enables controlled emptying of a syringe in a reliable, adjustable manner. By offering precise modulation of medication flow, it enhances the safety and efficiency of IV drug administration, reducing the workload on healthcare providers and improving patient outcomes.

This is achieved in that, the movable part slides on the fixed part and is attached to one part of a wire that runs along the fixed part in a loop over a first pulley, whereby the movable part moves in opposite directions relative to the fixed part when the wire runs around the pulley clockwise or counterclockwise.

Embodiments are recited in the dependent claims.

In another aspect the invention relates to a device as recited in claim 10.

An embodiment of the invention will now be described with reference to the figures, where:
Fig 1 shows the internal part in a device according to the invention.
Fig. 2a shows, in sideview, a syringe part.
Fig. 2b shows section c-c as indicated in figure 2a.
Fig. 3 shows a device provided with a food-pedal.

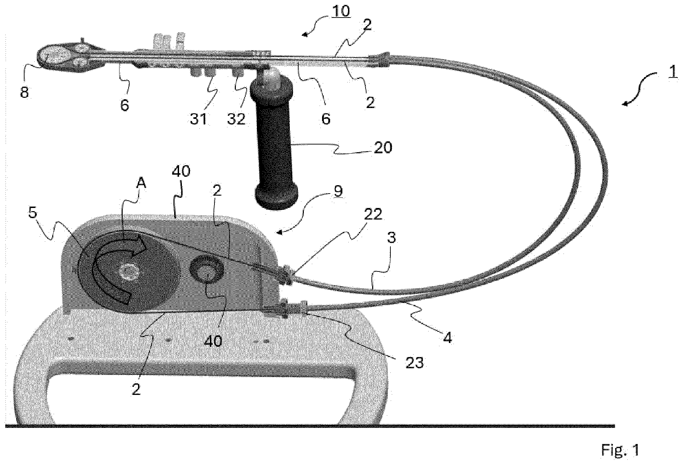

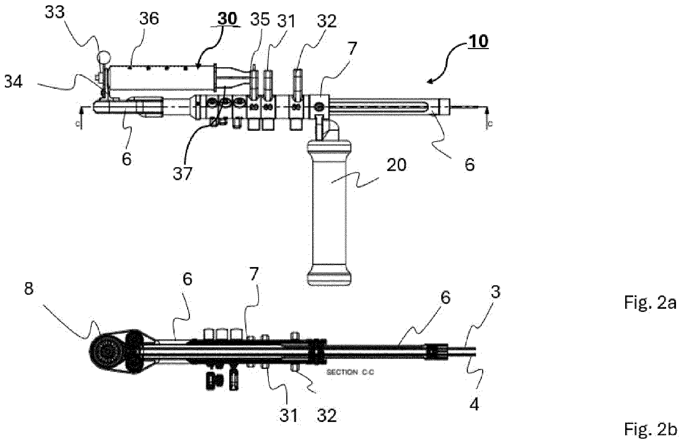

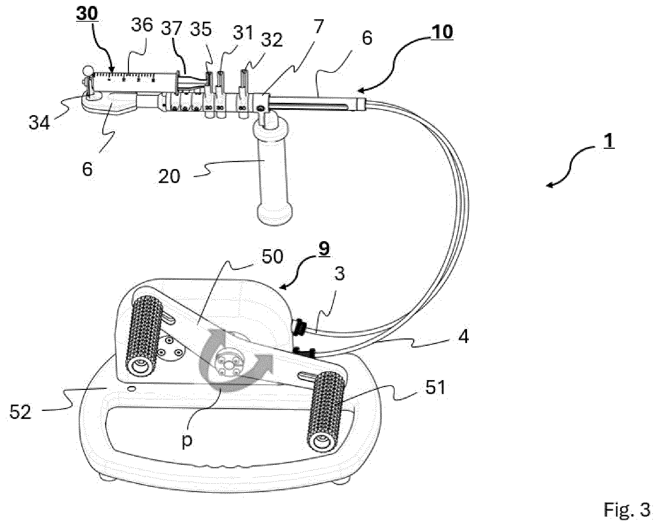

In figure 1, an embodiment of a device, 1 for emptying piston syringes is shown. The device 1 comprises a syringe section 10 and an activation section 9. The syringe section 10 includes a first part 6 for connection to a syringe cylinder (not shown in figure 1 but in figure 2 and 3) and a second part 7 for connection to a syringe piston. The two parts 6, 7 can move relative to each other towards or away from one another, and in the illustrated example, this is achieved by allowing the movable part 7 to slide on a slide piece on the fixed part 6.

Typically, but not necessarily, the two parts 6, 7 are designed as tubes that can slide over each other.

Thus, a syringe with a cylinder and piston attached to the activation section's first 6 and second part 7 can be emptied or filled by moving the syringe section's first 6 and second part 7 relative to each other. The activation section 9 and the syringe section 10 are connected by two hollow sheath parts 3 and 4, inside which a wire 2 runs. The wire 2 runs through the activation section over a pulley 5 so that the wire is moved by and with this pulley, which in this application is referred to as the second pulley. From the second pulley 5, the wire exits the activation section via two adjustment screws 21, 22 and continues through the sheath parts 3 and 4 into the syringe section 10.

In the syringe section, the wire runs along fixed part and passes the piece on the fixed part that the moveable part can slide on and further up to the tip of the fixed part 6, where it again passes over a pulley 8, which in the following is referred to as the first pulley.

Thus, the wire 2 may run continuously from the syringe section 10 through the two sheath parts 3 and 4 to the activation section 9. When the second pulley 5 is rotated clockwise, the wire will move out of the activation section's pulley 22 and further through sheath part 3, up along the lower side (as shown in the figure) of the syringe section, over the first pulley 8, and further down along the upper side of the syringe section, back to the second pulley 5 via sheath part 4 and adjustment screw 21.

According to the invention, the activation section's movable part 7 is attached to the part of the wire running through the upper or lower side of the fixed part 6. Thus, the activation section's movable part will move/slide to the left in the figure when the second pulley 5 is rotated clockwise as explained above, thereby allowing a syringe with its cylinder fixed to the fixed part 6 and its piston part 37 attached to the movable part to be emptied when the first pulley is rotated clockwise. Conversely, a syringe attached as described above can be filled when the second pulley is rotated counterclockwise.

In figure 2a, the syringe section with an inserted syringe 30 is shown. The syringe cylinder 36 is fixed in the syringe section 10 via its tip 33. For this fixation, the activation section's fixed part 6 is provided with a fixation device 34. On the movable part 7, rotatable adjustment devices 31, 32, 35 are provided, each of which can be attached to a syringe piston rod so that the amount displaced out of a fixed syringe can be adjusted by selecting the appropriate adjustment device for the desired amount and then rotating it up and securing it to the syringe's 30 piston rod 37.

In an embodiment not shown, the wire/cord solution shown in the figures can be replaced by a hydraulic mechanism, where a fluid runs in two pipes that are connected in a bend situated at the tip of the fixed part - where the first pulley is located. The two pipes run along the fixed and movable part like the wire. The movable part is moved in this embodiment by the fluid in one pipe. Such a solution can easily be made by a person skilled in the art of hydraulics and will therefore not be explained further. The actual movement of the fluid in the two pipes will typically be achieved with an activation unit comprising a hydraulic pump and the hydraulic pump can be mechanically driven via, for example, a tiltable foot pedal that is adapted and connected to the pipes so that it causes the fluid to run one way through the system when the foot pedal is tilted in one direction and the other way through the system when the foot pedal is tilted in the opposite direction. However, the hydraulic pump may also be electric driven.

Furthermore, the electric driven hydraulic pump may also be activatable by a switch situated at a tiltable foot pedal and such that the electric hydraulic pump starts and sends fluid in one direction through the two pipes when the foot pedal is tilted in a first direction and thereby hit and activates the switch. The electric hydraulic pump may also be activatable by a further switch situated such that it starts and sends the fluid in the opposite direction when the foot pedal is tilted in a direction opposite to the first direction and thereby hit and activates the further switch.

In figure 2b, the section CC shown in figure 2a is illustrated.

In figure 3, a system with both the syringe section 10 and the activation section 9 is shown. The activation section itself, in the illustrated embodiment, comprises a foot pedal 50 attached to the shaft 40 (shown in figure 1). The shaft 40 is connected to a transmission mechanism (not shown), which, for example, via a gearwheel, is further connected to the second pulley 5 so that the second pulley 5 rotates in either direction (clockwise or counterclockwise) when the foot pedal is tilted in each direction, as indicated by arrow P. In the illustrated embodiment, the foot pedal is provided with two identical adjustable footrests 51.

## Claims

1. A device for emptying or filling piston syringes (30) of the type comprising; a piston that can be pressed into a cylinder with a first end provided with a tip (33) for attaching a needle and a second end normally provided with a flange, the device comprising:
∘ a syringe section (10) with a fixed part (6) for attachment to a piston syringe cylinder (36, 33) and a movable part (7) for attachment to a piston syringe piston rod (37);
∘ wherein the movable part (7) can move in both directions relative to the fixed part (6) so that a syringe (30) fixed in the syringe section (10) can be emptied or filled by moving the movable part (7) relative to the fixed part (6), **characterized in that**, the movable part (7) slides on the fixed part (6) and is attached to one part of a wire (2) that runs along the fixed part (6) in a loop over a first pulley, whereby the movable part (7) moves in opposite directions relative to the fixed part (6) when the wire (2) is moved by an activation mechanism (9) around the pulley (8) in a clockwise or counterclockwise direction.

2. A device for emptying piston syringes according to claim 1, **characterized in that**, it comprises an electrically driven activation mechanism that can manipulate the wire to move it in a clockwise or counterclockwise direction.

3. A device for emptying piston syringes according to claim 1 or 2, **characterized in that** the movable part comprises a handle section (20).

4. A device for emptying piston syringes according to claims 1-3, **characterized in that**, the movable part (7) comprises adjustment devices, each of which can engage a piston part of a syringe (30), whose cylinder is fixed to the fixed part (6), whereby the amount emptied or filled into the syringe can be varied.

5. A device for emptying piston syringes according to claims 1-4, **characterized in that**, the device comprises an activation section (9) connected to the syringe section (10) with two hollow sheath cables (3, 4) through which the wire (2) runs so that a pull on the wire in one sheath cable causes the wire to run around the pulley (8) in one rotational direction, while a pull on the wire (2) in the other sheath cable causes the wire (2) to run around the pulley (8) in the opposite rotational direction.

6. A device for emptying piston syringes according to claim 5, **characterized in that**, the activation section (9) comprises a second pulley (5) over which the wire (2) runs in a loop so that in the activation section (9), it runs from one adjustment device over the second pulley (5) to the other adjustment device.

7. A device for emptying piston syringes according to claims 1-6, **characterized in that**, the wire is coated.

8. A device for emptying piston syringes according to claims 5-7, **characterized in that**, the second pulley (5) is connected to a tiltable pedal (50) in such a way that the second pulley (5) rotates one way when the foot pedal is tilted in one direction and the opposite way when the foot pedal is tilted in the other direction.

9. A device for emptying piston syringes according to claims 6-9, **characterized in that**, a gear mechanism is provided between the tilting pedal and the second pulley, changing the speed at which the second pulley rotates in relation to the speed at which the tilting pedal moves.

10. A device for emptying or filling piston syringes (30) of the type comprising; a piston that can be pressed into a cylinder with a first end provided with a tip (33) for attaching a needle and a second end normally provided with a flange, the device comprising:
∘ a syringe section (10) with a fixed part (6) for attachment to a piston syringe cylinder (36, 33) and a movable part (7) for attachment to a piston syringe piston rod (37);
∘ wherein the movable part (7) can move in both directions relative to the fixed part (6) so that a syringe (30) fixed in the syringe section (10) can be emptied or filled by moving the movable part (7) relative to the fixed part (6), **characterized in that** the movable part (7) and the fixed part (6) are mutually adapted such that the movement of the movable part occurs by it sliding on a slide piece adapted thereto on the fixed part (6), and that the movable part is moved by an actuator part which is hydraulically connected to fluid in one of two mutually connected pipes which run along the slide piece of the fixed part, such that the direction of movement of the hydraulic actuator part is dependent on the direction in which the fluid is pumped through the two connected pipes by a thereto adapted hydraulic pump.
